# DEMANDE DE BREVET EUROPEEN

(11) **EP 1 386 603 A1**
(43) Date de publication de la demande: **04.02.2004**
(21) Numéro de dépôt: 03291746.0
(22) Date de dépôt: 15.07.2003
(51) Int. Cl.: A61K 7/13

(54) **Utilisation de diamines comportant au moins un groupe quaternisé et de dialdéhydes pour la teinture des fibres kératiniques**

(30) Priorité: 01.08.2002 FR 0209803
(71) Demandeur: L'OREAL, 75008 Paris (FR)
(72) Inventeur: Plos, Grégory, 75011 Paris (FR)
(74) Mandataire: Dossmann, Gérard

(57) **Abrégé**

Composition de teinture des fibres kératiniques prête à l'emploi, préparée extemporanément, comprenant, dans un milieu approprié pour la teinture,
- au moins un dialdéhyde choisi parmi ceux de formule (I) ou (II) 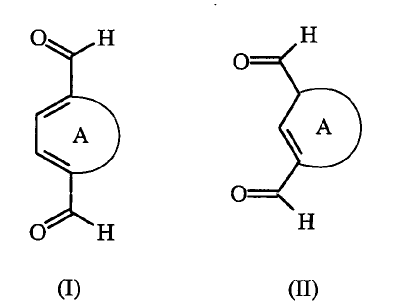 où A représente un groupe carbocyclique ou hétérocyclique portant éventuellement un ou plusieurs substituants halogéno, alkyle en C₁₋₆, hydroxyle, alcoxy en C₁₋₆, thiol, aldéhyde, acide carboxylique, nitro, sulfo ou hétérocyclique azoté, et
- au moins une diamine à groupes quaternisé(s),
des kits multi-compartiments contenant de tels composés, et des procédés de teinture des fibres kératiniques utilisant de tels kits ou une telle composition.

## Description

La présente invention concerne l'utilisation de diamines à groupe(s) quaternisé(s) et de dialdéhydes pour la teinture des fibres kératiniques, une composition de teinture prête à l'emploi contenant au moins une telle diamine quaternisée et au moins un dialdéhyde, des procédés de teinture des fibres kératiniques utilisant ces composés et des kits multicompartiments permettant de les mettre en oeuvre.

La modification de la couleur des fibres kératiniques telles que les cheveux, implique souvent, en plus de l'apport d'un colorant, une étape préalable ou simultanée consistant à éclaircir les fibres. Un tel éclaircissement se fait généralement par destruction partielle ou totale de la mélanine à l'aide de produits oxydants tels que l'eau oxygénée, en milieu basique. Les agents chimiques responsables de la décoloration de la mélanine attaquent toutefois également la kératine des cheveux dont les propriétés cosmétiques et mécaniques peuvent se dégrader fortement en cas de décolorations répétées.

La demanderesse, dans le cadre de ses recherches visant à mettre au point de nouvelles techniques de teinture préservant la structure chimique des fibres kératiniques, a découvert, avec surprise, qu'il était possible de déposer, sur des cheveux assez foncés, des couleurs relativement plus claires sans utilisation d'agents oxydants, c'est-à-dire sans altération chimique de la structure des cheveux grâce à la réaction entre au moins un dialdéhyde et au moins une diamine cationique quaternisée décrits tous deux plus en détail ci-après. Ces deux réactifs polymérisent en effet *in situ* et forment à la surface des cheveux un dépôt de polymère coloré, bien visible sur des cheveux foncés et qui, grâce à son excellente affinité pour la kératine, résiste bien à l'élimination par des shampooings.
La demanderesse a également découvert qu'il était possible de fixer sur le dépôt polymérique à groupes quaternisés ainsi formé, toutes sortes de principes actifs cosmétiques anioniques, et notamment des colorants anioniques, ce qui permet d'améliorer les propriétés cosmétiques des cheveux traités ou d'apporter d'autres reflets au premier dépôt coloré.

La présente invention a par conséquent pour objet des compositions prêtes à l'emploi pour la teinture des fibres kératiniques, en particulier des fibres kératiniques humaines et plus particulièrement des cheveux, préparées extemporanément, comprenant, dans un milieu approprié pour la teinture,
- au moins un dialdéhyde choisi parmi ceux de formule (I) ou (II) ci-après, et
- au moins une diamine à groupe(s) quaternisé(s) de formule (III) ou (IV) ci-après.

L'invention a également pour objet des kits multi-compartiments permettant de préparer les compositions prête à l'emploi ci-dessus, ainsi que différents procédés de teinture utilisant les diamines de formule (I) et (II) et dialdéhydes quaternisés de formule (III) et (IV).

L'invention a encore pour objet l'utilisation d'au moins une diamine quaternisée de formule (III) ou (IV) et d'au moins un dialdéhyde de formule (I) ou (II) tels que définis ci-après, pour la teinture des fibres kératiniques humaines, et plus particulièrement des cheveux

Les dialdéhydes utilisés selon la présente invention pour la teinture des fibres kératiniques, sont choisis parmi ceux répondant aux formules (I) et (II) suivantes : 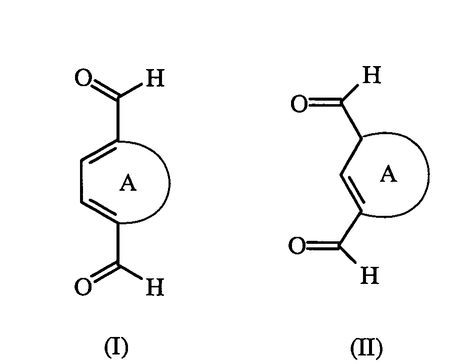 où A représente
- un groupe carbocyclique, monocyclique ou polycyclique à cycles condensés ou non-condensés, aromatique ou non aromatique, comprenant de 6 à 50 atomes de carbone, ou
- un groupe hétérocyclique, monocyclique ou polycyclique à cycles condensés ou non-condensés, aromatique ou non-aromatique, comprenant de 5 à 30 chaînons, éventuellement associé, via un bras de liaison, à un groupe carbocyclique, monocyclique ou polycyclique à cycles condensés ou non-condensés, comprenant de 5 à 30 atomes de carbone,
les hétéroatomes du groupe hétérocyclique étant choisis parmi l'azote, le soufre, l'oxygène et/ou le phosphore, et les groupes carbocycliques ou hétérocycliques portant éventuellement un ou plusieurs substituants halogéno, alkyle en C₁₋₆, hydroxyle, alcoxy en C₁₋₆, thiol, aldéhyde, acide carboxylique, nitro, sulfo ou hétérocyclique azoté.

Parmi ces dialdéhydes, on préfère en particulier ceux de formule (I) où A représente un noyau 1,4-phénylène non-substitué ou un noyau 1,4-phénylène portant de 1 à 4 substituants halogéno, alkyle en C₁₋₆, hydroxyle, alcoxy en C₁₋₆, thiol, aldéhyde, acide carboxylique, nitro, sulfo ou hétérocyclique azoté.
On peut citer à titre d'exemples de tels dialdéhydes préférés le téréphtaldéhyde (CAS 623-27-8), le 2,3,5,6-tétraméthyl-benzène-1,4-dicarboxyaldéhyde (CAS 7072-01-7) et le 2,5-diméthoxy-benzène-1,4-dicarboxyaldéhyde (CAS 7310-97-6).

Comme dialdéhyde de formule (II) on peut citer ceux où A représente un noyau 1,3-phénylène non-substitué ou un noyau 1,3-phénylène portant de 1 à 4 substituants choisis parmi les groupes halogéno, alkyle en C₁₋₆, hydroxyle, alcoxy en C₁₋₆, thiol, aldéhyde, acide carboxylique, nitro, sulfo ou hétérocyclique azoté.
Des exemples de tels composés de formule (II) englobent par exemple le 5-méthylbenzène-1,3-dicarboxaldéhyde (CAS 1805-67-0), le 5-formylsalicylaldéhyde (CAS 3328-70-9), le 2,6-diformyl-4-méthylphénol (CAS 7310-95-4), le 4-méthoxyisophtalaldéhyde commercialisé par la société Maybridge, le 2,4,6-triméthylisophtalaldéhyde commercialisé par la société Acros, le 2-hydroxybenzène-1,3,5-tricarbaldéhyde commercialisé par la société Maybridge, et le 2,6-diformyl-4-chlorophénol (CAS 32596-43-3).
Comme indiqué ci-dessus, le noyau A peut également représenter un groupe polycyclique. Des exemples de dialdéhydes aromatiques à noyau polycyclique utilisables selon l'invention sont le naphtalène- 1,4-dicarboxaldéhyde (CAS 38153-01-4) et le 9,10-anthracènedicarboxaldéhyde (CAS 7044-91-9).

Les diamines cationiques quaternisées que l'on fait réagir selon la présente invention avec les dialdéhydes décrits ci-dessus pour former des polymères colorés à la surface des fibres kératiniques, peuvent être des diamines cationiques monomères portant un seul groupe quaternisé ou des diamines oligomères ou polymères portant plusieurs groupes quaternisés et résultant de la condensation de deux ou plusieurs diamines cationiques monomères.
Ces deux types de diamines cationiques quaternisées, utilisables selon la présente invention, correspondent respectivement aux formules (III) et (IV) : 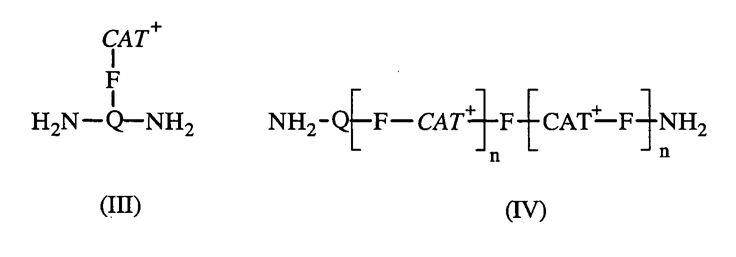 dans lesquelles
chaque n représente, indépendamment, un nombre entier compris entre 1 et 20,
chaque F représente, indépendamment, un groupe alkylène en C₁₋₁₄, linéaire ou ramifié, comportant éventuellement un ou plusieurs hétéroatomes choisi parmi N, O et S,
chaque Q représente, indépendamment,
- un groupe carbocyclique, monocyclique ou polycyclique à cycles condensés ou non-condensés, aromatique ou non aromatique, comprenant de 6 à 50 atomes de carbone, ou
- un groupe hétérocyclique, monocyclique ou polycyclique à cycles condensés ou non-condensés, aromatique ou non-aromatique, comprenant de 5 à 30 chaînons, éventuellement associé, via un bras de liaison, à un groupe carbocyclique, monocyclique ou polycyclique à cycles condensés ou non-condensés, comprenant de 5 à 30 atomes de carbone, ou
- une chaîne aliphatique en C₆₋₅₀, ramifiée ou non, éventuellement substituée,
   les groupes carbocycliques ou hétérocycliques ou la chaîne aliphatique portant éventuellement un ou plusieurs substituants halogéno, alkyle en C₁₋₆, hydroxyle, alcoxy en C₁₋₆, thiol, aldéhyde, acide carboxylique, nitro, sulfo ou hétérocyclique azoté,
chaque *CAT*⁺ représente, indépendamment,
- un groupe ammonium quaternaire monovalent de formule -N⁺R₃ ou un groupe ammonium quaternaire divalent de formule -N⁺R₂- où chaque R représente indépendamment un groupe alkyle en C₁₋₆,
- ou un hétérocycle aromatique azoté, monovalent ou divalent, avec de 5 ou 10 chaînons, comprenant un seul atome d'azote quaternisé par F ou par un groupe alkyle en C₁₋₆ éventuellement mono- ou polyhydroxylé, et éventuellement un ou plusieurs atome(s) d'azote non-quaternisé(s).

On peut citer à titre d'exemples de groupes hétérocycliques aromatiques azotés quaternisés (CAT⁺) les groupes imidazolium, pyridinium, benzothiazolium, triazolium et pyrazolium.

On peut citer à titre d'exemple de diamine monomère à groupe quaternisé les composés de formule générale (IIIa) ci-dessous : 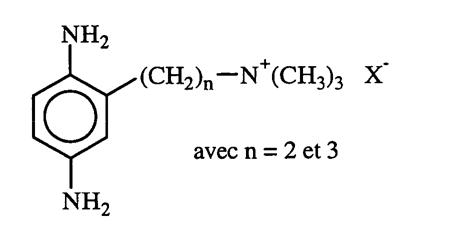 cités dans le brevet US 4 975 092.

Une diamine dimère qui donne d'excellents résultats de teinture est le composé de formule (IVa) :

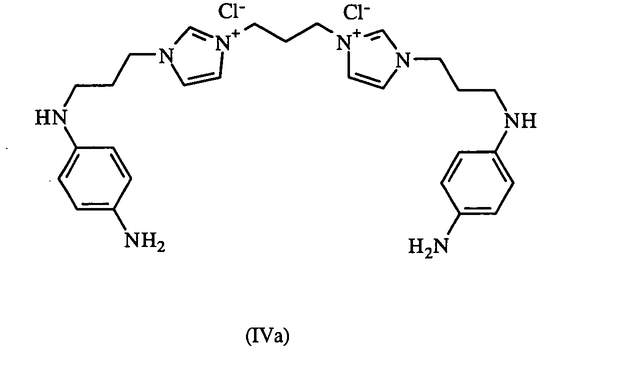

Ce composé est décrit dans la demande internationale WO 99/03834.

Les polymères formés *in situ* sur la fibre kératinique par réaction entre les dialdéhydes et les diamines à groupe(s) quaternisé(s) décrits ci-dessus, ont une charge positive élevée. Cette charge leur confère une excellente affinité pour la kératine des cheveux qui a une charge globale anionique. Les colorations obtenues ont de ce fait une excellente résistance, en particulier au lavage.

La forte charge cationique des polymères colorés obtenus par réaction entre les dialdéhydes et les diamines quaternisées de la présente invention présente encore d'autres avantages. En effet, il est possible de fixer sur le polymère et donc sur les cheveux, n'importe quel principe actif anionique susceptible de protéger les cheveux ou de modifier leurs propriétés, tels que des filtres UV, des agents tensioactifs, des polymères, des colorants ou des antioxydants.

Dans un mode de réalisation préféré de l'invention, le principe actif cosmétique anionique est un colorant direct acide. Ce colorant direct, qui peut être présent dans la composition prête à l'emploi ou bien être appliqué sur les cheveux après celle-ci, permet de renforcer l'intensité de la coloration ou de faire varier la nuance de la coloration obtenue. Il est ainsi possible d'obtenir un large spectre de couleurs et de nuances.

Les colorants directs acides sont choisis parmi ceux connus dans la technique, et de préférence parmi ceux portant au moins une fonction acide carboxylique ou acide sulfonique. De tels colorants sont décrits dans l'ouvrage Color Index, International Third Edition.
Les principales familles au sein desquelles on peut trouver des colorants anioniques particulièrement adaptés sont les suivantes : les colorants azoïques ou méthiniques, les colorants anthraquinoniques et les colorants triarylméthaniques.

Les compositions prêtes à l'emploi contiennent le dialdéhyde, la diamine à groupe(s) quaternisé(s) et, éventuellement, le principe actif cosmétique anionique à une concentration allant, pour chacun de ces composés, d'environ 0,01 à 30 % en poids, de préférence d'environ 0,05 à 20 % en poids, rapportée au poids total de la composition prête à l'emploi.

La polycondensation *in situ* entre le dialdéhyde et la diamine nécessite le mélange extemporané, c'est-à-dire immédiatement avant emploi, de ces deux composants actifs qui doivent être conditionnés et conservés dans des récipients séparés afin d'éviter une polymérisation précoce.

La présente invention a donc également pour objet des kits multi-compartiments contenant le dialdéhyde et la diamine dans des récipients séparés. Le principe actif cosmétique anionique, présent facultativement, peut être introduit dans un de ces récipients ou dans les deux, ou bien être conservé dans un troisième récipient séparé des deux premiers.

Par conséquent dans un premier mode de réalisation, le kit multi-compartiment pour la teinture des fibres kératiniques de la présente invention, comprend :
- un premier compartiment (a) contenant au moins un dialdéhyde de formule (I) ou (II) tel que défini ci-dessus, et
- un deuxième compartiment (b) contenant au moins une diamine à groupe(s) quaternisé(s) de formule (III) ou (IV) telle que définie ci-dessus.

Lorsque l'on souhaite immobiliser un ou plusieurs principes actifs anioniques sur le polymère cationique coloré formé par réaction entre le dialdéhyde et la diamine, le kit multi-compartiments décrit ci-dessus, peut comprendre en outre un troisième compartiment (c) contenant ce ou ces principes actifs cosmétiques anioniques, ou bien au moins un des deux compartiments (a) et (b) peut contenir, en plus du dialdéhyde ou de la diamine, ledit ou lesdits principes actifs cosmétiques anioniques.
Dans ce dernier cas, et en particulier lorsque le principe actif est un colorant direct acide, ce colorant est ajouté de préférence dans le compartiment contenant la diamine quaternisée. Lorsque le principe actif anionique est exempt de groupes amines susceptibles de réagir avec le dialdéhyde, il peut toutefois également être conservé dans le même récipient que le dialdéhyde.

Les procédés de teinture des fibres kératiniques utilisant les composés décrits ci-dessus peuvent être des procédés comprenant une seule étape d'application d'un produit (procédé en un temps), deux étapes d'application d'un produit (procédé en deux temps), voire même trois étapes d'application d'un produit (procédé en trois temps).

Le procédé en un temps comprend plus précisément les étapes suivantes consistant
- à préparer, immédiatement avant emploi, une composition prête à l'emploi telle que décrite ci-dessus,
- à appliquer cette composition prête à l'emploi, préparée extemporanément, sur les fibres kératiniques,
- à laisser la composition en contact avec les fibres kératiniques pendant un temps de pose suffisant pour obtenir la coloration recherchée, et
- à rincer les fibres kératiniques.

La composition prête à l'emploi peut, bien entendu, être préparée de différentes manières à partir des kits de teinture décrits ci-dessus. Concrètement elle est préparée, par exemple,
- par mélange d'une première composition (a) contenant au moins un dialdéhyde de formule (I) ou (II) ci-dessus et d'une deuxième composition (b) contenant au moins une diamine à groupe(s) quaternisé(s) de formule (III) ou (IV) ci-dessus, ou
- par mélange d'une première composition (a) contenant au moins un dialdéhyde de formule (I) ou (II) ci-dessus, d'une deuxième composition (b) contenant au moins une diamine à groupe(s) quaternisé(s) de formule (III) -ou (IV) ci-dessus, et d'une troisième composition (c) contenant au moins un principe actif cosmétique anionique,
   les desdites compositions (a) et/ou (b) pouvant contenir en outre au moins un principe actif cosmétique anionique.

On peut également envisager d'appliquer ledit principe actif cosmétique anionique dans une étape séparée, consécutive à celle de l'application de la composition prête à l'emploi contenant le dialdéhyde et la diamine qui forment le polymère coloré.

Lorsque le principe actif est un colorant direct acide, ce mode de réalisation permet par exemple d'appliquer ledit colorant seulement sur une partie des fibres traitées avec la première composition et d'apporter ainsi, par endroits, un reflet différent ou une couleur plus intense. Ce procédé en deux temps permet également l'application séparée de différents colorants directs anioniques en des zones différentes ce qui permet de créer une grande variété de motifs colorés.

Concrètement, un procédé de teinture des fibres kératiniques en deux temps de l'invention comprend les étapes suivantes consistant
- à appliquer sur les fibres kératiniques une première composition prête à l'emploi, préparée extemporanément, contenant au moins un dialdéhyde de formule (I) et (II) et au moins une diamine à groupe(s) quaternisé(s) de formule (III) et (IV), et à laisser cette composition en contact avec les fibres kératiniques pendant un temps de pose suffisant pour obtenir la coloration recherchée,
- à procéder éventuellement à un rinçage des fibres kératiniques avec de l'eau,
- à appliquer sur une partie ou la totalité des fibres kératiniques une composition (c) contenant au moins un actif cosmétique anionique et à laisser cette composition en contact avec les fibres kératiniques pendant un temps de pose compris entre 5 minutes et 1 heure, de préférence entre 5 et 30 minutes, puis
- à rincer les fibres kératiniques.

Dans les procédés en un temps et en deux temps décrits ci-dessus, l'application de la composition prête à l'emploi sur les fibres kératiniques doit se faire le plus rapidement possible, c'est-à-dire moins de 10 minutes et de préférence moins de 5 minutes après mélange des différentes compositions décrites ci-dessus.
Le temps de pose suffisant pour obtenir la coloration recherchée est généralement compris entre 5 minutes et 1 heure, de préférence entre 5 et 30 minutes.

Un autre procédé de teinture des fibres kératiniques en deux temps selon l'invention comprend les étapes suivantes consistant
- à appliquer sur les fibres kératiniques une première composition (a) contenant au moins un dialdéhyde de formule (I) ou (II) ci-dessus, et à laisser cette composition en contact avec les fibres kératiniques pendant un temps de pose compris entre 5 minutes et 1 heure, de préférence entre 5 et 30 minutes,
- à procéder éventuellement à un rinçage des fibres kératiniques avec de l'eau,
- à appliquer sur les fibres kératiniques une deuxième composition (b) contenant au moins une diamine à groupe(s) quaternisé(s) de formule (III) ou (IV) ci-dessus, et à laisser cette composition en contact avec les fibres kératiniques pendant un temps de pose compris entre 5 minutes et 1 heure, de préférence entre 5 et 30 minutes; puis
- à rincer les fibres kératiniques.

Enfin, un procédé de teinture des fibres kératiniques en trois temps selon l'invention comprend les étapes suivantes consistant :
- à appliquer sur les fibres kératiniques une première composition (a) contenant au moins un dialdéhyde de formule (I) ou (II) ci-dessus, et à laisser cette composition en contact avec les fibres kératiniques pendant un temps de pose compris entre 5 minutes et 1 heure, de préférence entre 5 et 30 minutes,
- à procéder éventuellement à un rinçage des fibres kératiniques avec de l'eau,
- à appliquer sur les fibres kératiniques une deuxième composition (b) contenant au moins une diamine à groupe(s) quaternisé(s) de formule (III) ou (IV) ci-dessus, et à laisser cette composition en contact avec les fibres kératiniques pendant un temps de pose compris entre 5 minutes et 1 heure, de préférence entré 5 et 30 minutes
- à procéder éventuellement à un rinçage des fibres kératiniques avec de l'eau, puis
- à appliquer sur une partie ou sur la totalité des fibres kératiniques une composition (c) contenant au moins un actif cosmétique anionique et à laisser cette composition en contact avec les fibres kératiniques pendant un temps de pose compris entre 5 minutes et 1 heure, de préférence entre 5 et 30 minutes, et enfin
- à rincer les fibres kératiniques.

On peut, bien entendu, également envisager une variante du procédé de teinture en deux temps et du procédé de teinture en trois temps décrits ci-dessus, dans laquelle l'ordre d'application des compositions (a) et (b), est inversé.

Ces deux derniers procédés, bien qu'ils soient relativement complexes, présentent l'avantage considérable de permettre (lorsque ledit principe actif n'est pas un colorant direct anionique) la teinture des cheveux sans utilisation d'une composition colorée. En effet, les différentes compositions, appliquées séparément à la suite l'une de l'autre, sont incolores et ne laisseront pas de taches, une couleur se développant seulement *in situ* lors de l'application de la deuxième composition. Ceci permet de réduire considérablement le risque de tacher les vêtements, les mains ou d'autres objets susceptibles de venir en contact avec la composition de teinture.
Un autre avantage d'un tel procédé où l'application du dialdéhyde et celle de la diamine se font dans des étapes séparées, réside dans le fait qu'il n'exige pas une vitesse d'exécution élevée car une réaction colorée précoce entre ces deux réactifs, qui viennent en contact seulement après application sur les fibres kératiniques, est impossible.

La réaction colorée entre le dialdéhyde et la diamine quaternisée qui est à la base des procédés de teinture des fibres kératiniques de la présente invention peut être accélérée grâce à l'application de chaleur, par exemple au moyen d'un rayonnement, par exemple d'un rayonnement infrarouge, ou d'air chaud, sec ou humide, provenant par exemple d'un sèche-cheveux ou d'un générateur de vapeur. Cet apport de chaleur pendant le temps de pose se fait généralement de manière à augmenter la température jusqu'à une valeur au plus égale à 80 °C, de préférence au plus égale à 60 °C.

Le milieu approprié pour la teinture des compositions de teinture de la présente invention est de préférence un milieu aqueux constitué d'eau et pouvant contenir avantageusement des solvants organiques acceptables sur le plan cosmétique, parmi lesquels on peut citer par exemple les alcools tels que l'éthanol, l'alcool isoproylique, l'alcool benzylique et l'alcool phényléthylique, les glycols ou éthers de glycol tels que les éthers monométhylique, monoéthylique et monobutylique d'éthylènglycol, le propylèneglycol ou ses éthers tels que le monométhyléther de propylèneglycol, le butylèneglycol, le dipropylèneglycol ainsi que les alkyléther de diéthylèneglycol comme par exemple le monoéthyléther ou le monobutyléther de diéthylèneglycol. Les solvants peuvent alors être présents en des concentrations allant d'environ 0,5 à 20 % en poids, de préférence d'environ 2 à 10 % en poids par rapport au poids total de la composition.
Les compositions de teinture prêtes à l'emploi et les compositions (a), (b) et (c) décrites ci-dessus peuvent contenir en outre des principes actifs cosmétiques différents des principes anioniques décrits ci-dessus ou des adjuvants de formulation bien connus en cosmétique et en particulier dans le domaine de la teinture des fibres kératiniques.
Les principes actifs cosmétiques différents des principes actifs anioniques décrits ci-dessus peuvent être choisis parmi les vitamines ou provitamines, les saccharides, les oligosaccharides, les polysaccharides hydrolysés ou non, modifiés ou non, les acides aminés, les oligopeptides, les peptides, les protéines hydrolysées ou non, modifiées ou non, les polyacides aminés, les acides et alcools gras ramifiés ou non, les esters gras, les cires animales, végétales ou minérales, les huiles végétales, minérales ou synthétiques, les céramides et les pseudo-céramides, les acides organiques hydroxylés, les filtres UV, les agents antipelliculaires, les agents régulateurs de séborrhée, les agents apaisants, les agents tensioactifs cationiques, les polymères cationiques, les polymères amphotères, les silicones organomodifiées ou non, les polymères associatifs de type non-ionique, cationique ou amphotère comportant au moins une chaîne grasse.
Les adjuvants de formulation sont choisis par exemple parmi les agents épaississants, les agents d'ajustement et de fixation du pH, les agents conservateurs, les séquestrants, les opacifiants, les agents réducteurs ou antioxydants, les parfums et les agents tensioactifs non-cationiques.
Bien entendu, l'homme du métier veillera à choisir le ou les éventuels composés complémentaires ci-dessus de manière à ce que les propriétés avantageuses intrinsèques des compositions de teinture prête à l'emploi selon l'invention ne soient pas altérées par la ou les adjonctions envisagées.

Le pH de la composition prête à l'emploi selon l'invention va de préférence de 4 à 11, et en particulier de 6 à 10. Il peut être ajusté à la valeur souhaitée par addition d'agents acidifiants ou alcalinisants ou de tampons bien connus dans la technique de teinture des fibres kératiniques.

La composition de teinture selon l'invention peut se présenter sous des formes diverses telles qu'un liquide, une crème ou un gel.

La présente invention est illustrée ci-après à l'aide d'exemples de réalisation qui n'ont toutefois aucun caractère limitatif.

### Exemple 1

### Teinture de cheveux blancs

### 1) Procédé en deux temps

Pour les essais de teinture sur cheveux blancs, on a préparé les formulations 1 à 3 ci-après. La formulation 1 contenant à la fois un dialdéhyde et une diamine à groupes quaternisés a été préparée immédiatement avant application sur les cheveux (moins de 10 minutes avant application). Les formulations 2 et 3 contenant chacune un colorant direct anionique sont stables et n'ont pas nécessité une préparation extemporanée.

| | **Formulation 1** | **Formulation 2** | **Formulation 3** |
|---|---|---|---|
| téréphtaldéhyde | 3.10⁻³ mole | - | - |
| diamine aromatique de formule (IVa) | 6.10⁻³ mole | - | - |
| Acid Blue 147 | - | 0,5 g | - |
| Acid Red 66 | - | - | 0,5 g |
| hydroxyéthylcellulose (PM : 720 000) | 0,72 g | 0,72 g | 0,72 g |
| NIPA®^{a)} | 0,06 g | 0,06 g | 0,06 g |
| Oramix® CG110^{b)} | 5 g | 5 g | 5 g |
| alcool benzylique | 4 g | 4 g | 4 g |
| polyéthylèneglycol (8 OE) | 6 g | 6 g | 6 g |
| tampon borate (pH 9) (50 mM) | 50 g | 50 g | 50 g |
| eau distillée | q.s.p. 100 g | q.s.p. 100 g | q.s.p. 100 g |

| | | | |
|---|---|---|---|
| a) Mélange de p-hydroxybenzoates de méthyle, butyle, éthyle, propyle et isobutyle commercialisé par la société CLARIANT | | | |
| b) (alkyle en C_{8/10})polyglycoside (mélange 50/50) à 60 % en solution aqueuse commercialisé par la société SEPPIC | | | |

On a appliqué la formulation 1 avec un rapport de bain de 10 sur des mèches de cheveux naturels et permanentés à 90% de blancs, on a laissé reposer pendant 30 minutes à température ambiante, on a rincé, puis séché.

Sur les cheveux ainsi traités, on a appliqué dans les mêmes conditions (rapport de bain : 10, temps de pose : 30 min., température ambiante) la formulation 2 ou la formulation 3, puis on a rincé les cheveux, on les a lavés avec un shampooing usuel et on les a séchés.

On a observé visuellement les colorations suivantes :

| | cheveux naturels | cheveux permanentés |
|---|---|---|
| Formulation 1 | jaune mat | jaune mat |
| Formulation 1 + Formulation 2 | vert | vert foncé |
| Formulation 1 + Formulation 3 | cuivré orangé | cuivré |

### Exemple 2

### Teinture de cheveux blancs

### Procédé en un temps

On a préparé immédiatement avant application sur les cheveux (moins de 10 minutes avant application) la formulation 4 suivante

| | **Formulation 4** |
|---|---|
| téréphtaldéhyde | 3.10⁻³ mole |
| diamine aromatique de formule (IVa) | 6.10⁻³ mole |
| Acid Blue 147 | 0,5 g |
| 2-amino-2-méthyl-1-propanol | q.s.p. pH 9 |
| eau distillée | q.s.p. 100 g |

On a appliqué la formulation 4 avec un rapport de bain de 10 sur des mèches de cheveux naturels et permanentés à 90 % de blancs, on a laissé reposer pendant 30 minutes à température ambiante, on a rincé à l'eau, on a lavé avec un shampooing usuel, puis on a séché.

L'intensité de coloration a été évaluée par colorimétrie selon le système CIELAB à l'aide d'un colorimètre CM2002 de Minolta (illuminant D65, angle d'observation : 10 °, composantes spéculaires exclues).
Le système de notation CIELAB définit un espace colorimétrique dans lequel chaque couleur est définie par 3 paramètres (L*, a* et b*) :
- le paramètre L* reflète *la clarté* de la couleur, la valeur de L* étant égale à 0 pour le noir et égale à 1 pour le blanc absolu. Plus la valeur de L* est élevée, moins la coloration est intense.
- le paramètre a* correspond à l'axe du couple antagoniste vert-rouge et le paramètre b* à l'axe du couple antagoniste bleu-jaune.
Le tableau ci-dessous montre les paramètres L*, a* et b* des mèches de cheveux naturels et de cheveux permanentés après la montée de la couleur :

| | **cheveux naturels** | | | **cheveux permanentés** | | |
|---|---|---|---|---|---|---|
| | **L*** | **a*** | **b*** | **L*** | **a*** | **b*** |
| Formulation 6 | 33,31 | -8,00 | 13,82 | 27,96 | -7,76 | 9,50 |

### Exemple 3

### Coloration sur cheveux châtains

En appliquant 3 fois le procédé en un temps de l'exemple 2 sur des cheveux châtains, on a obtenu, sans décoloration préalable, une couleur verte visible sur ce fond.

## Revendications

1. Composition de teinture des fibres kératiniques, en particulier des fibres kératiniques humaines et plus particulièrement des cheveux, prête à l'emploi, préparée extemporanément, comprenant, dans un milieu approprié pour la teinture,
• au moins un dialdéhyde choisi parmi ceux de formule (I) ou (II) 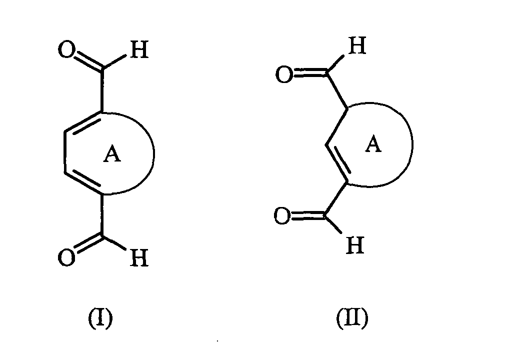
où A représente
• un groupe carbocyclique, monocyclique ou polycyclique à cycles condensés ou non-condensés, aromatique ou non aromatique, comprenant de 6 à 50 atomes de carbone, ou
• un groupe hétérocyclique, monocyclique ou polycyclique à cycles condensés ou non-condensés, aromatique ou non-aromatique, comprenant de 5 à 30 chaînons, éventuellement associé, via un bras de liaison, à un groupe carbocyclique, monocyclique ou polycyclique à cycles condensés ou non-condensés, comprenant de 5 à 30 atomes de carbone,
les hétéroatomes du groupe hétérocyclique étant choisis parmi l'azote, le soufre, l'oxygène et/ou le phosphore, et les groupes carbocycliques ou hétérocycliques portant éventuellement un ou plusieurs substituants halogéno, alkyle en C₁₋₆, hydroxyle, alcoxy en C₁₋₆, thiol, aldéhyde, acide carboxylique, nitro, sulfo ou hétérocyclique azoté, et
• au moins une diamine comportant au moins un groupe quaternisé, de formule (III) ou (IV) : 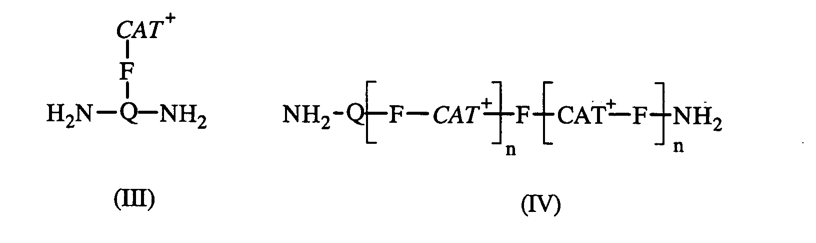
dans lesquelles
chaque n représente, indépendamment, un nombre entier compris entre 1 et 20,
chaque F représente, indépendamment, un groupe alkylène en C₁₋₁₄, linéaire ou ramifié, comportant éventuellement un ou plusieurs hétéroatomes choisi parmi N, O et S,
chaque Q représente, indépendamment,
• un groupe carbocyclique, monocyclique ou polycyclique à cycles condensés ou non-condensés, aromatique ou non aromatique, comprenant de 6 à 50 atomes de carbone, ou
• un groupe hétérocyclique, monocyclique ou polycyclique à cycles condensés ou non-condensés, aromatique ou non-aromatique, comprenant de 5 à 30 chaînons, éventuellement associé, via un bras de liaison, à un groupe carbocyclique, monocyclique ou polycyclique à cycles condensés ou non-condensés, comprenant de 5 à 30 atomes de carbone, ou
• une chaîne aliphatique en C₆₋₅₀, ramifiée ou non, éventuellement substituée,
les groupes carbocycliques ou hétérocycliques ou la chaîne aliphatique portant éventuellement un ou plusieurs substituants halogéno, alkyle en C₁₋₆, hydroxyle, alcoxy en C₁₋₆, thiol, aldéhyde, acide carboxylique, nitro, sulfo ou hétérocyclique azoté,
chaque *CAT*^{*+*} représente, indépendamment,
• un groupe ammonium quaternaire monovalent de formule -N⁺R₃ ou un groupe ammonium quaternaire divalent de formule -N⁺R₂- où chaque R représente indépendamment un groupe alkyle en C₁₋₆,
• ou un hétérocycle aromatique azoté, monovalent ou divalent, avec de 5
ou 10 chaînons, comprenant un seul atome d'azote quaternisé par F ou par un groupe alkyle en C₁₋₆ éventuellement mono- ou polyhydroxylé, et éventuellement un ou plusieurs atomes d'azote non-quaternisés.

2. Composition selon la revendication 1, **caractérisée par le fait que** le dialdéhyde est un dialdéhyde de formule (I) ou (II) où A représente respectivement un noyau 1,4-phénylène ou 1,3-phénylène non-substitués ou un noyau 1,4-phénylène ou 1,3-phénylène portant de 1 à 4 substituants choisis parmi les groupes halogéno, alkyle en C₁₋₆, hydroxyle, alcoxy en C₁₋₆, thiol, aldéhyde, acide carboxylique, nitro, sulfo ou hétérocyclique azoté.

3. Composition selon la revendication 2, **caractérisée par le fait que** le dialdéhyde est choisis parmi le téréphtaldéhyde, le 2,3,5,6-tétraméthylbenzène-1,4-dicarboxaldéhyde, le 2,5-diméthoxybenzène-1,4-dicarboxaldéhyde, le 5-méthylbenzène-1,3-dicarboxaldéhyde, le 5-formylsalicylaldéhyde, le 2,6-diformyl-4-méthylphénol, le 4-méthoxyisophtalaldéhyde, le 2,4,6-triméthylisophtalaldéhyde, le 2-hydroxybenzène-1,3,5-tricarbaldéhyde et le 2,6-diformyl-4-chlorophénol.

4. Composition selon la revendication 1, **caractérisée par le fait que** le dialdéhyde est choisi parmi le naphtalène-1,4-dicarboxaldéhyde et le 9,10-anthracènedicarboxaldéhyde.

5. Composition selon l'une quelconque des revendications précédentes, **caractérisée par le fait que** le dialdéhyde est présent en une concentration allant de 0,01 % à 30 % en poids, de préférence de 0,05 % à 20 % en poids, rapporté au poids total de la composition prête à l'emploi.

6. Composition selon l'une quelconque des revendications précédentes, **caractérisée par le fait que** la diamine à groupe(s) quaternisé(s) est présente en une concentration allant de 0,01 à 30 % en poids, de préférence de 0,05 à 20 % en poids, rapporté au poids total de la composition prête à l'emploi.

7. Composition selon l'une quelconque des revendications précédentes, **caractérisée par le fait qu'**elle contient en outre un ou plusieurs principes actifs cosmétiques anioniques.

8. Composition selon la revendication 7, **caractérisée par le fait que** le ou les principes actifs anioniques sont choisis parmi les colorants directs portant au moins un groupe acide carboxylique ou acide sulfonique.

9. Composition selon l'une des revendications 7 et 8, **caractérisée par le fait que** le ou les principes actifs cosmétiques anioniques sont présents en une concentration allant de 0,01 à 30 % en poids, de préférence de 0,05 à 20 % en poids, rapporté au poids total de la composition.

10. Composition selon l'une quelconque des revendications précédentes, **caractérisée par le fait qu'**elle a un pH allant de 4 à 11, de préférence de 6 à 10.

11. Kit multi-compartiments pour la teinture des fibres kératiniques, **caractérisé par le fait qu'**il comprend
• un premier compartiment (a) contenant au moins un dialdéhyde de formule (I) ou (II) tel que défini dans la revendication 1, et
• un deuxième compartiment (b) contenant au moins une diamine à groupe(s) quaternisé(s) de formule (III) ou (IV) telle que définie dans la revendication 1.

12. Kit multi-compartiments pour la teinture des fibres kératiniques selon la revendication 11, **caractérisé par le fait qu'**il comprend en outre un troisième compartiment (c) contenant au moins un principe actif cosmétique anionique.

13. Kit multi-compartiments pour la teinture des fibres kératiniques selon la revendication 11 ou 12, **caractérisé par le fait qu'**au moins un des deux compartiments (a) et (b) contient en outre au moins un principe actif cosmétique anionique.

14. Kit multi-compartiments pour la teinture des fibres kératiniques selon l'une des revendications 12 et 13, **caractérisé par le fait que** le ou les principes actifs anioniques sont choisis parmi les colorants directs portant au moins un groupe acide carboxylique ou acide sulfonique.

15. Procédé de teinture des fibres kératiniques comprenant les étapes suivantes consistant
• à préparer, immédiatement avant emploi, une composition prête à l'emploi selon l'une quelconque des revendications 1 à 10,
• à appliquer cette composition prête à l'emploi, préparée extemporanément, sur les fibres kératiniques,
• à laisser la composition en contact avec les fibres kératiniques pendant un temps de pose suffisant pour obtenir la coloration recherchée, et
• à rincer les fibres kératiniques.

16. Procédé de teinture des fibres kératiniques selon la revendication 15, **caractérisé par le fait que** l'on prépare la composition prête à l'emploi par mélange
• d'une première composition (a) contenant au moins un dialdéhyde de formule (I) ou (II) tel que défini dans la revendication 1,
• d'une deuxième composition (b) contenant au moins une diamine à groupe(s) quaternisé(s), de formule (III) ou (IV) telle que définie dans la revendication 1, et
• d'une troisième composition (c) contenant au moins un principe actif cosmétique anionique.

17. Procédé de teinture des fibres kératiniques comprenant les étapes suivantes consistant
• à appliquer sur les fibres kératiniques une première composition prête à l'emploi, préparée extemporanément, selon l'une quelconque des revendications 1 à 6 et à laisser cette composition en contact avec les fibres kératiniques pendant un temps de pose suffisant pour obtenir la coloration recherchée,
• à procéder éventuellement à un rinçage des fibres kératiniques avec de l'eau,
• à appliquer sur une partie ou la totalité des fibres kératiniques une composition (c) contenant au moins un actif cosmétique anionique et à laisser cette composition en contact avec les fibres kératiniques pendant un temps de pose compris entre 5 minutes et 1 heure, de préférence entre 5 et 30 minutes, puis
• à rincer les fibres kératiniques.

18. Procédé de teinture des fibres kératiniques selon l'une quelconque des revendications 15 à 17, **caractérisé par le fait que** le temps de pose est compris entre 5 minutes et 1 heure, de préférence entre 5 et 30 minutes.

19. Procédé de teinture des fibres kératiniques comprenant les étapes suivantes consistant :
• à appliquer sur les fibres kératiniques une première composition (a) contenant au moins un dialdéhyde de formule (I) ou (II) tel que défini dans la revendication 1, et à laisser cette composition en contact avec les fibres kératiniques pendant un temps de pose compris entre 5 minutes et 1 heure, de préférence entre 5 et 30 minutes,
• à procéder éventuellement à un rinçage des fibres kératiniques avec de l'eau,
• à appliquer sur les fibres kératiniques une deuxième composition (b) contenant au moins une diamine à groupe(s) quaternisé(s) de formule (III) ou (IV) telle que définie dans la revendication 1, et à laisser cette composition en contact avec les fibres kératiniques pendant un temps de pose compris entre 5 minutes et 1 heure, de préférence entre 5 et 30 minutes, et enfin
• à rincer les fibres kératiniques.

20. Procédé de teinture des fibres kératiniques comprenant les étapes suivantes consistant :
• à appliquer sur les fibres kératiniques une première composition (a) contenant au moins un dialdéhyde de formule (I) ou (II) tel que défini dans la revendication 1, et à laisser cette composition en contact avec les fibres kératiniques pendant un temps de pose compris entre 5 minutes et 1 heure, de préférence entre 5 et 30 minutes,
• à procéder éventuellement à un rinçage des fibres kératiniques avec de l'eau,
• à appliquer sur les fibres kératiniques une deuxième composition (b) contenant au moins une diamine à groupe(s) quaternisé(s), de formule (III) ou (IV) telle que définie dans la revendication 1, et à laisser cette composition en contact avec les fibres kératiniques pendant un temps de pose compris entre 5 minutes et 1 heure, de préférence entre 5 et 30 minutes
• à procéder éventuellement à un rinçage des fibres kératiniques avec de l'eau, puis
• à appliquer sur une partie ou la totalité des fibres kératiniques une composition (c) contenant au moins un actif cosmétique anionique et à laisser cette composition en contact avec les fibres kératiniques pendant un temps de pose compris entre 5 minutes et 1 heure, de préférence entre 5 et 30 minutes, et enfin
• à rincer les fibres kératiniques.

21. Procédé de teinture des fibres kératiniques selon la revendication 19 ou 20, **caractérisé par le fait que** l'on inverse l'ordre d'application des compositions (a) et (b).

22. Procédé de teinture des fibres kératiniques selon l'une des revendications 15 à 18 et 20, **caractérisé par le fait que** le ou les principes actifs anioniques sont choisis parmi les colorants directs portant au moins un groupe acide carboxylique ou acide sulfonique.

23. Procédé de teinture des fibres kératiniques selon l'une quelconque des revendications 15 à 22, **caractérisé par le fait que** l'on applique de la chaleur pendant le temps de pose de manière à augmenter la température jusqu'à une valeur au plus égale à 80 °C, de préférence au plus égale à 60 °C.

24. Utilisation d'au moins une diamine quaternisée de formule (III) ou (IV) et d'au moins un dialdéhyde de formule (I) ou (II) tels que définis dans l'une quelconque des revendications 1 à 4, pour la teinture des fibres kératiniques humaines, et plus particulièrement des cheveux.
